# FASCICULE DE BREVET EUROPEEN

(11) **EP 1 030 617 B1**
(45) Date de publication et mention de la délivrance du brevet: **21.05.2003**
(21) Numéro de dépôt: 98954075.2
(22) Date de dépôt: 16.11.1998
(51) Int. Cl.: A61B 19/00

(54) **DISPOSITIF DE MANIPULATION D'INSTRUMENTS CHIRURGICAUX**
VORRICHTUNG ZUR BEDIENUNG VON CHIRURGISCHEN INSTRUMENTEN
DEVICE FOR POSITIONING SURGICAL INSTRUMENTS

(30) Priorité: 14.11.1997 EP 97870183
(43) Date de publication de la demande: 30.08.2000
(73) Titulaire: Medsys S.A., 5030 Gembloux (BE)
(72) Inventeur: ANDRE, Jacques, B-1410 Waterloo (BE); MANGEZ, Jean, B-5020 Namur (BE); MOREELS, Xavier, B-5030 Gembloux (BE); POLET, Roland, B-1150 Bruxelles (BE)
(74) Mandataire: Van Malderen, Joelle
(86) Numéro de dépôt international: BE9800177
(87) Numéro de publication internationale: WO99025267

(56) Documents cités:
- EP-A- 0 293 227
- EP-A- 0 293 760
- EP-A- 0 752 237
- WO-A-94/26167
- WO-A-95/10389
- WO-A-95/16396
- US-A- 5 441 042
- US-A- 5 540 649
- US-A- 5 571 072

## Description

### Objet de l'invention

La présente invention se rapporte à un dispositif qui permet la manipulation, c'est-à-dire le positionnement et le déplacement, d'instruments chirurgicaux, tels qu'un endoscope, lors d'une opération chirurgicale.

### Etat de la technique

Il existe depuis de nombreuses années des aides se présentant sous la forme de dispositifs mécaniques pouvant porter des instruments chirurgicaux destinés à aider le chirurgien au cours d'opérations.

Des améliorations ont été apportées à ces aides, de telle sorte que ces dispositifs peuvent se déplacer et éventuellement actionner ces instruments chirurgicaux, et en particulier les scopes.

Ces dispositifs sont essentiellement destinés à libérer les mains du chirurgien afin qu'il n'ait plus à interrompre un acte pour déplacer par exemple le scope ou à devoir confier la manipulation de celui-ci à un assistant.

Néanmoins, la plupart des différents dispositifs connus n'offrent pas une précision dans le positionnement de l'instrument et une stabilité de maintien et/ou de déplacement suffisantes pour les applications dans le domaine médical telles que les actes chirurgicaux.

En particulier, on connaît un dispositif de stabilisation de l'endoscope tel que publié dans le document US-5184601, qui comprend une console indépendante munie d'un robot à axes multiples qui permet la réalisation de mouvements de rotation autour d'axes verticaux afin de couvrir un plan horizontal (XY) parallèle au patient. Ce dispositif présente les inconvénients de nécessiter une combinaison de plusieurs mouvements pour déplacer un scope selon les directions et orientations souhaitées par le chirurgien, et d'encombrer le champ d'action du chirurgien par la présence de bras se déplaçant dans le plan horizontal (XY) au-dessus du patient.

On connaît également par le document WO94/26167 un dispositif de positionnement destiné à porter un instrument pour des applications de chirurgie laparoscopique, qui prévoit un centre de rotation sphérique dudit instrument, ce centre étant constitué par le point de pénétration dans le ventre du patient, par exemple. Néanmoins, on observe que ce dispositif ne permettra pas la réalisation d'une translation selon l'axe de l'instrument, mouvement qui doit être rendu possible pour l'utilisation d'un scope.

Le document WO95/16396 décrit un dispositif de manipulation d'un instrument chirurgical à l'aide de plusieurs bras successifs qui effectuent des rotations autour d'axes verticaux ainsi que proposé ci-dessus, et qui, par un calcul complexe et détaillé des différents mouvements des actionneurs, permettra d'obtenir un déplacement incrémental de la tête du scope. Cependant, ces mouvements sont obtenus par trois changements de coordonnées cartésiennes successifs, ce qui rend la visualisation des mouvements relativement complexe. Afin de prévoir une aide décisionnelle à la manipulation de l'instrument, une commande à la voix peut également être proposée. Cette commande à la voix ne permet néanmoins pas la réalisation de petits déplacements rapides et/ou à cadence élevée.

Les documents EP-A-0293760 et US-A-5 540 649 se rapportent au même dispositif de manipulation, qui comprend un pied vertical subissant un premier mouvement de rotation verticale. Le second mouvement est une rotation selon un axe orthogonal à l'axe vertical. Ceci permet de réaliser un déplacement de tout le dispositif au-dessus de patient même, limitant ainsi le champ d'action du chirurgien.

### Buts de l'invention

La présente invention vise à proposer un dispositif de manipulation d'instruments chirurgicaux qui soit d'une conception relativement simple et d'un encombrement le plus réduit possible.

Un autre but de la présente invention vise à proposer un dispositif qui permette de libérer autant que possible les mains du chirurgien.

Un autre but de la présente invention vise à réduire autant que possible l'encombrement du champ d'action du chirurgien.

Un autre but de la présente invention vise à proposer un dispositif qui permette une combinaison de mouvements simples reproduisant les trois mouvements fondamentaux d'exploration du champ visuel.

Un autre but de la présente invention vise à proposer un dispositif de commande qui est simple et très peu encombrant, de telle sorte qu'il permette la manipulation d'autres instruments.

D'autres buts et avantages apparaîtront à la lecture du mémoire descriptif qui suit.

### Principaux éléments caractéristiques de la présente invention

La présente invention se rapporte à un dispositif de manipulation d'un instrument chirurgical composé d'un élément support fixe associé à au moins :
- un premier élément mobile articulé à l'élément support fixe autour d'un premier axe essentiellement parallèle au plan dans lequel est disposé le patient, générant ainsi un premier mouvement;
- un second élément mobile articulé à l'extrémité du premier élément autour d'un deuxième axe essentiellement parallèle au plan dans lequel est disposé le patient, générant ainsi un second mouvement;
- un troisième élément déformable fixé par un côté à l'extrémité du deuxième élément mobile et susceptible d'effectuer un troisième mouvement de rotation autour d'un troisième axe;
- un élément de préhension fixé du côté opposé à celui du deuxième élément à l'élément déformable;
- un dispositif d'accrochage d'un instrument chirurgical à l'élément de préhension; et
- des moyens d'action permettant d'effectuer des mouvements de rotation autour des différents axes.

Ceci signifie de manière générale que les premier et second mouvements s'effectuent autour d'axes essentiellement horizontaux en particulier lorsque le plan dans lequel est disposé le patient est lui-même horizontal. De préférence, les deux axes horizontaux sont parallèles l'un à l'autre, et leur projection dans le plan du patient sont orthogonales à l'axe du patient.

De préférence, les premier et deuxième éléments mobiles se présentent sous la forme de bras articulés tandis que l'élément déformable se présente sous la forme d'un quadrilatère déformable et articulé.

Le deuxième élément mobile articulé présente deux points d'articulation dont l'un peut coulisser dans une glissière et dont l'autre peut être bloqué ou libre.

Selon une forme d'exécution particulière, l'élément de préhension est disposé dans une glissière solidaire de l'élément déformable.

L'élément support est fixé à un rail solidaire de la table d'opération à l'aide d'un dispositif de fixation.

Selon une première forme d'exécution, ce dispositif de fixation comprend au moins deux caissons qui effectuent un premier mouvement orthogonal au lit du patient et un second mouvement longitudinal parallèle au lit du patient. Une vis permet de réaliser le mouvement du premier caisson dans le second caisson de manière à obtenir le premier mouvement qui est orthogonal au lit du patient. Le second mouvement qui est longitudinal est réalisé à l'aide d'un troisième caisson qui comprend des roulements qui coulissent sur une glissière. La fixation du dispositif est réalisée lorsque l'on serre une ou plusieurs vis en clamant le rail entre les clames et un élément adéquat.

Selon une autre forme d'exécution, le mouvement longitudinal est réalisé à l'aide de plusieurs, et de préférence quatre roulements utilisant le rail de la table d'opération comme glissière, une clame inférieure qui permet, au moyen d'une molette, d'immobiliser l'ensemble sur ledit rail.

Le dispositif de fixation peut être inversé par rotation autour d'un axe vertical et est ajusté par glissement le long du rail de fixation solidaire de la table d'opération.

En outre, selon une forme d'exécution préférée, le manipulateur comprend un dispositif de commande constitué d'un mini-clavier à au moins six touches pour deux ou trois doigts, et dont la partie intérieure est de forme triangulaire. Ce dispositif de commande est complètement autonome et comporte un émetteur qui transmet les commandes au boîtier principal, cet émetteur se présentant sous la forme d'un petit boîtier de formé triangulaire.

### Description des figures

- La figure 1: représente une vue en perspective de la table d'opération sur laquelle le patient sera couché et à laquelle est fixé le dispositif de manipulation selon la présente invention.
- Les figures 2 et 3: représentent une vue en coupe latérale du dispositif de manipulation d'instruments chirurgicaux selon la présente invention pour deux applications d'interventions chirurgicales.
- Les figures 4 et 5: représentent des vues en coupe frontale du dispositif tel que représenté aux figures 2 et 3.
- La figure 6: représente une vue en coupe latérale correspondant à la figure 2 mais permettant de visualiser les différents mouvements du manipulateur.
- Les figures 7 et 8: représentent une vue en coupe latérale et une vue en coupe par le dessus d'un dispositif de fixation permettant de fixer le manipulateur au lit du patient selon une première forme d'exécution.
- Les figures 9, 10 et 11: représentent une seconde forme d'exécution du dispositif de fixation du manipulateur au lit du patient.

### Description de plusieurs formes d'exécution préférées de l'invention

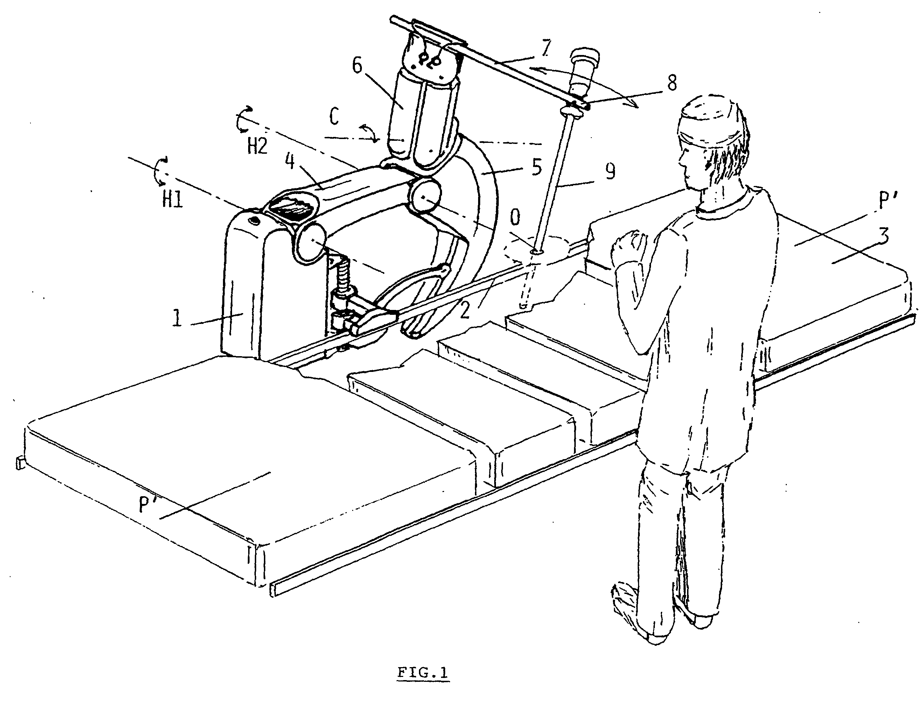

Le dispositif de manipulation selon la présente invention et représenté à la figure 1 en perspective est solidaire du lit sur lequel le patient va se coucher.

Ce manipulateur présente l'avantage particulier qu'il possède un champ de déplacement tel que l'on peut l'appliquer à deux grandes familles d'interventions chirurgicales : d'une part les interventions chirurgicales gynécologiques et d'autre part les interventions chirurgicales abdominales. Il suffit, pour passer d'un champ à l'autre, de retourner simplement l'appareil par rapport à un plan vertical qui est perpendiculaire à l'axe selon lequel est couché le patient. Ceci signifie que l'appareil est bien sûr réversible et qu'il suffit de l'orienter selon la position avant ou arrière par rapport au patient.

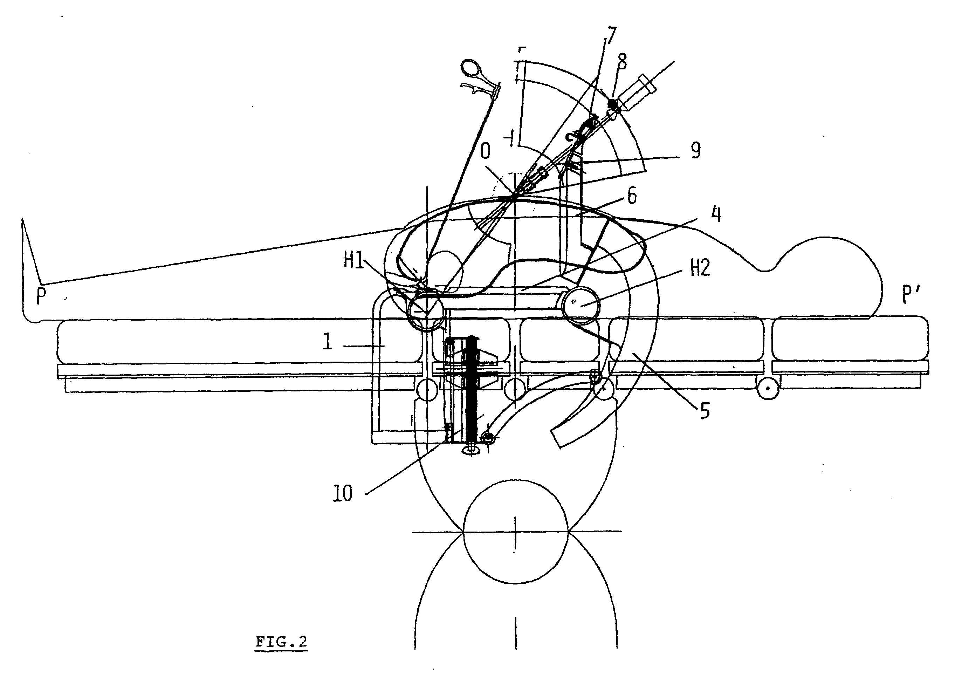

La figure 2 représente plus précisément le dispositif disposé de manière à permettre une intervention de type gynécologique. Ceci signifie essentiellement que l'endoscope pénétrera dans le patient dans une position telle que celui-ci permet d'obtenir une visualisation du champ où le chirurgien devra intervenir.

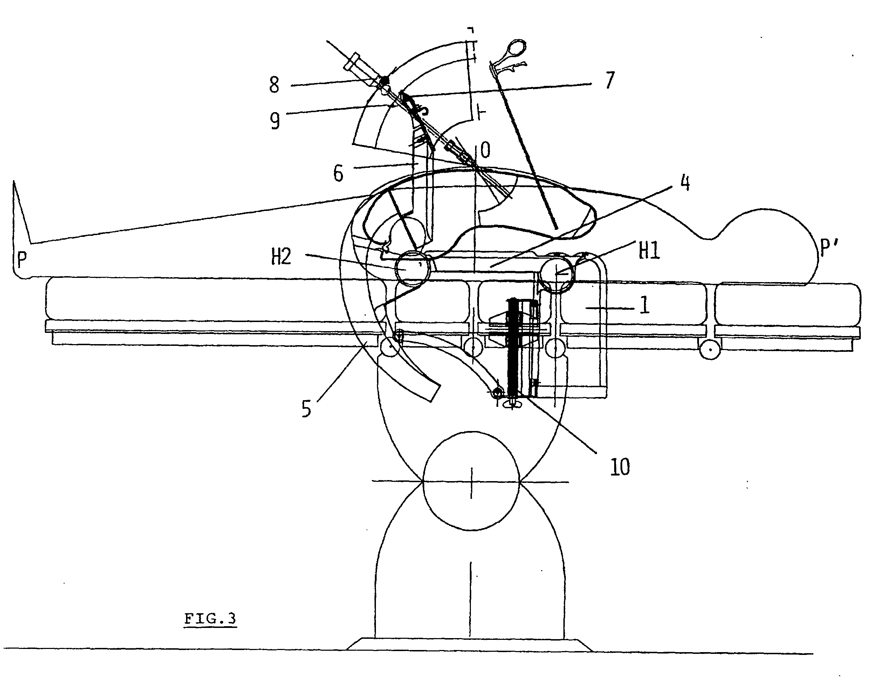

La figure 3 représente le dispositif disposé de manière à permettre une intervention de type abdominal. Dans ce cas, l'endoscope pénétrera dans le patient dans une position telle que celle-ci permet une visualisation vers la tête du patient.

D'autres types d'interventions chirurgicales comme des interventions orthopédiques peuvent également être envisagées.

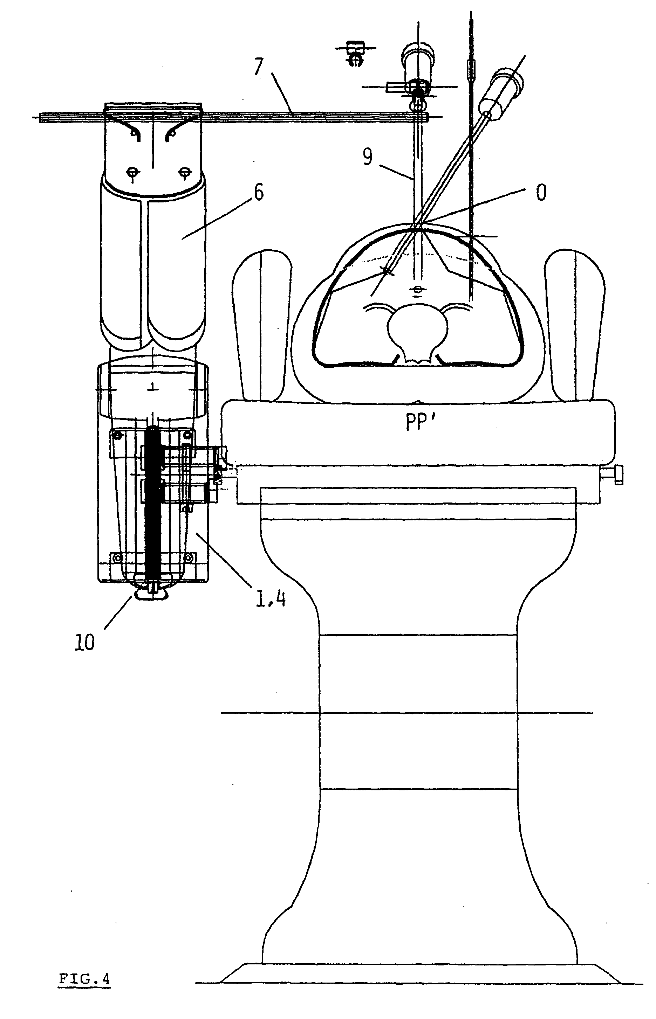

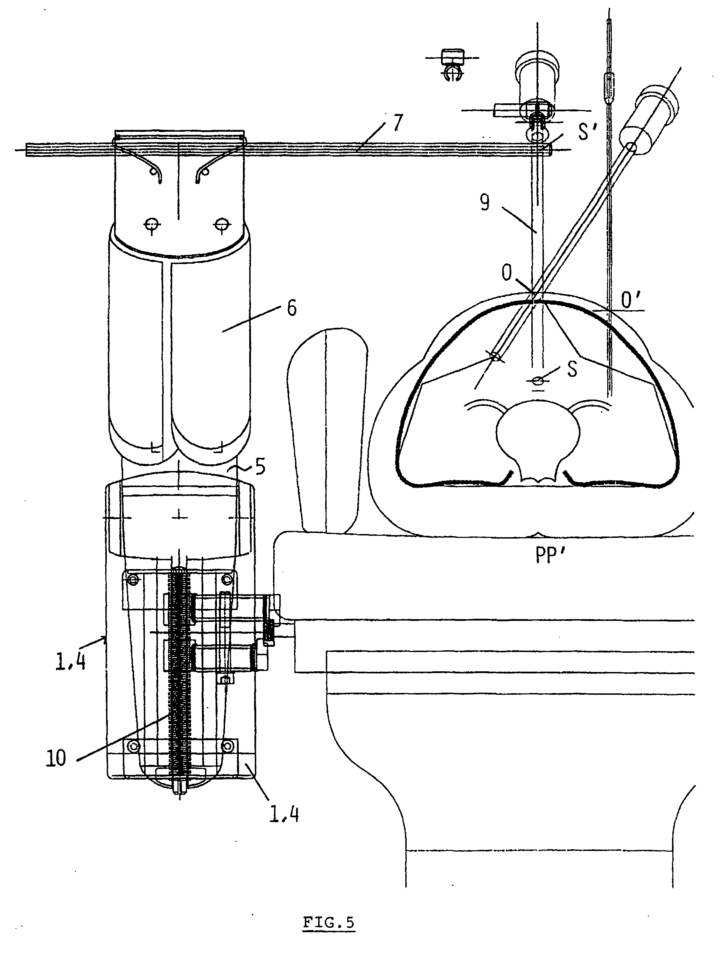

Dans les figures 1 à 6, le corps du patient est disposé selon un axe PP' avec la tête du patient dirigée vers P'. O représente le point de pénétration de l'instrument dans le ventre du patient. Ce point représente en général le nombril du patient.

Ainsi qu'il apparaît dans les diverses figures, ce manipulateur comprend au moins les éléments suivants :
- un support (1) (après ajustement en hauteur et longitudinalement) sera fixé sur un rail (2) solidaire de la table d'opération (3), ce support fixé étant associé à :
   - un premier bras mobile (4) articulé au support fixé autour d'un axe essentiellement horizontal H1 perpendiculaire au plan vertical passant par l'axe longitudinal du patient POP' ;
   - un deuxième bras mobile (5) articulé à l'extrémité du premier autour d'un deuxième axe essentiellement horizontal H2, également perpendiculaire au même plan POP' ;
   - un élément déformable (6) fixé par un côté à l'extrémité du deuxième bras (5) et susceptible d'effectuer un mouvement de rotation autour d'un axe C;
   - un bras de préhension (7) horizontal fixé au côté opposé du quadrilatère (6) ;
   - un dispositif d'accrochage (8) d'un instrument (9) au bras de préhension (7) ;
   - des actionneurs qui permettent d'exécuter des mouvements de rotation autour des différents axes mentionnés H1, H2 et C;
   - des moyens de pilotage et de synchronisation de ces mouvements; et
- une commande placée dans le creux de la main du praticien, lui permettant, au moyen de plusieurs touches, d'accéder aux différents mouvements.

Le manipulateur permet donc de déplacer un instrument (9) par des mouvements autour de deux axes (H1 et H2) de rotation parallèles entre eux et au lit du patient ainsi qu'autour d'un troisième axe (C) de rotation.

On observe que selon la présente invention, le premier mouvement de rotation ne se fait pas autour d'un axe perpendiculaire à la table d'opération et plus particulièrement vertical. Ceci permet de ce fait de dégager de manière particulièrement avantageuse le champ d'action du chirurgien.

La combinaison des mouvements permet d'obtenir les trois mouvements fondamentaux d'exploration du champ visuel, tels que représentés à la table 1.

**Table 1**

| **Mouvements de l'oeil** | **Mouvement donné au scope** | | |
|---|---|---|---|
| | **Direction** | **Sens** | **Symbole (+ sens)** |
| Accommodation (proche/lointain) | Zoom | in/out | ζ (i/o) |
| Rotation verticale (haut / bas) | Rotation | haut/bas | ρ (h/b) |
| Rotation horizontale (gauche / droite) | Latéral | gauche/droite | λ (g/d) |

Les mouvements produits correspondront donc à :
ζ (i/o) : déplacement suivant l'axe du scope (SS')
ρ (h/b) : rotation du scope dans le plan POP' autour de O
λ (g/d) : rotation du scope dans le plan S'OO' autour de O

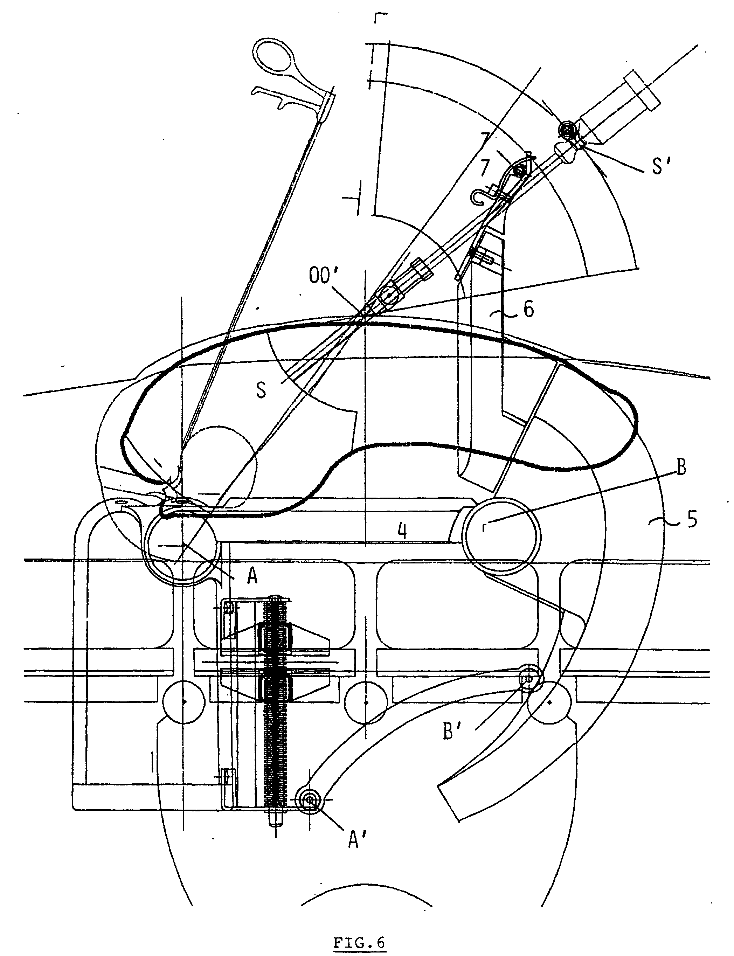

De préférence, le deuxième bras (5) se présente lui-même sous la forme d'un des côtés (BB') d'un parallélogramme articulé (ABB'A') tel que représenté plus en détail à la figure 6. L'articulation (B') peut coulisser suivant une glissière (51) solidaire du bras (5) ; la position par rapport au bras (5) et la longueur du bras (BB') peuvent donc varier.
Deux cas de figure peuvent se présenter :
- l'articulation (B) est bloquée : les bras et éléments (5, 6 et 7) sont donc simplement entraînés par la rotation du support (1) autour de l'axe H1, et le mouvement de S' et S est donc voisin de ρ;
- l'articulation (B) est libre, mais la position de (B') dans sa glissière est bloquée par un solénoïde (52) : dans ce cas, le mouvement du parallélogramme (6) entraîné par la rotation du support (1) autour de l'axe H1 induit un déplacement du bras de préhension (7), et donc de S' et S, voisin de ζ.

Cette exécution permet d'assurer les mouvements ρ et ζ au moyen d'un seul servomoteur.

Le bras de préhension (7) est toujours horizontal. Ceci permet de limiter au maximum l'encombrement créé par celui-ci dans le champ d'action du chirurgien. Il est relié au mécanisme et au lit du patient par le côté opposé au chirurgien.

L'instrument de chirurgie (9), tel qu'un endoscope, est relié au bras de préhension (7) par l'intermédiaire d'un joint passif (non motorisé) de manière à reproduire l'image que ce dernier perçoit et qu'elle soit toujours orientée correctement par rapport aux axes de référence du local, c'est-à-dire que les directions verticales doivent apparaître comme verticales sur le moniteur. La rotation du second bras (5) et la déformation du parallélogramme articulé (6) sont assurées par deux servomoteurs incorporés au bras (5).

La fixation du scope (9) au bras de préhension (7) en S' peut être assurée au moyen d'un joint passif constitué au moins de deux pièces articulées suivant l'axe (j) perpendiculaire à l'axe (d) du bras de préhension (7) et à SS'.

La pièce tenant le scope est fixée à ce dernier dans une position telle que le repère de verticalité de l'image soit dans le plan vertical passant par (j). De cette façon, l'image du scope apparaîtra toujours verticale pour un observateur fictif situé à la tête du patient et regardant parallèlement à l'axe PP'.

La pièce accrochée au bras de préhension (7) en S' est clipsée sur ce bras de préhension (7) de façon à pouvoir tourner librement autour de l'axe (d). Cette pièce est conçue pour pouvoir être déclipsée du bras de préhension (7) par simple action sur un petit levier.

En variante, cet ensemble peut être valablement réalisé en une seule pièce d'une matière élastique.

Le dispositif de commande du manipulateur peut être constitué d'un mini-clavier à six touches pour deux ou trois doigts (auriculaire et annulaire, et majeur pour la version à trois doigts) placé dans le creux de la main du praticien au moyen d'une pince adéquate entourant la main du praticien, caractérisée en ce que la partie intérieure (dans le creux de la main), de forme triangulaire, s'inscrive dans le "trapèze" de la main, entre l'éminence thénar et le pli des quatre phalanges, ne gênant ainsi aucunement les mouvements de la main.

De préférence, le bras de préhension (7) de l'instrument (9) peut être ajusté dans une glissière (71) de façon à disposer S' et donc S de manière qu'ils soient situés dans le plan POP'. Ceci constitue l'ajustement du point neutre de S en début d'opération. Grâce à cette particularité, on peut adapter le manipulateur à l'un de l'autre type d'opération : abdominale ou gynécologique (voir figures 1 et 2) simplement en le tournant de 180° de dispositif de fixation et en inversant puis ajustant le bras de préhension (7) dans sa glissière (71).

De manière générale, le manipulateur est fixé à un rail (2) lui-même solidaire de la table d'opération (3) à l'aide d'un dispositif de fixation (100).

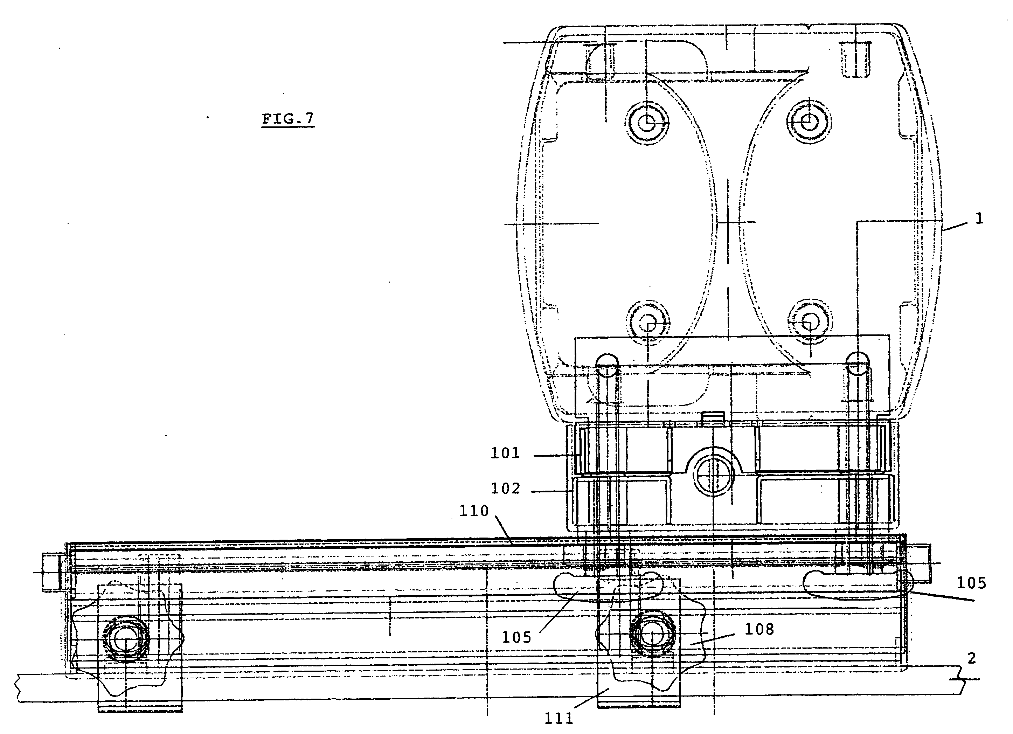

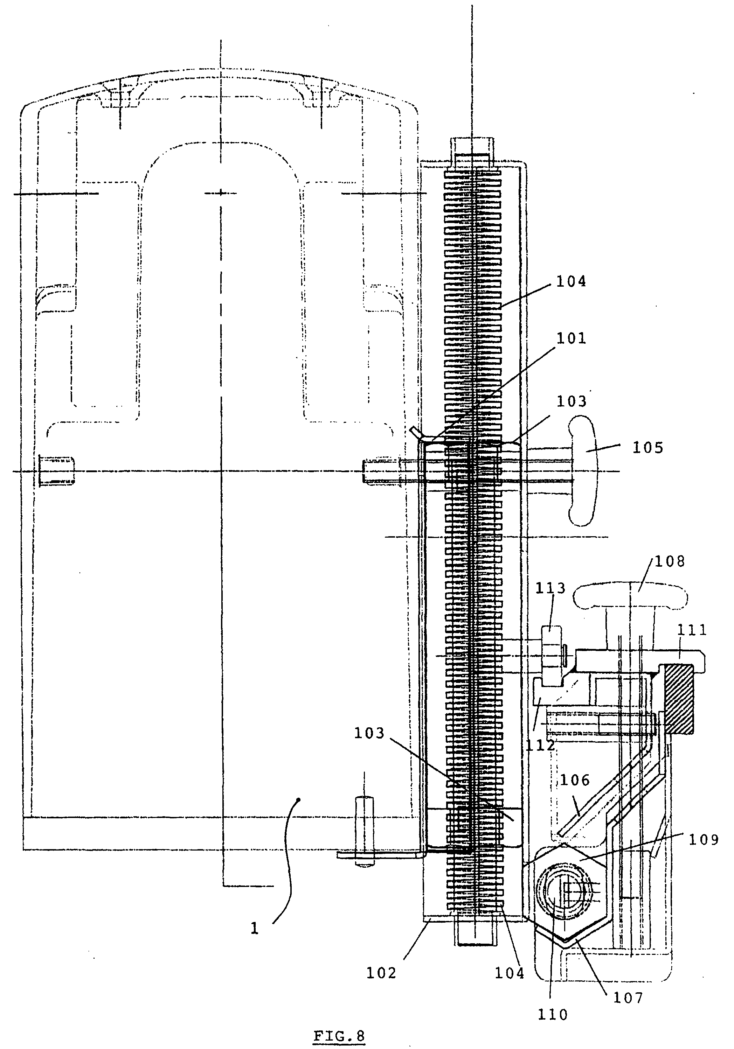

Selon une première forme d'exécution, un dispositif de fixation (100) du manipulateur à la table d'opération est représenté aux figures 7 et 8. Ce dispositif (100) permet donc d'ajuster précisément la position du support (1) longitudinalement (parallèlement au rail (2) de la table d'opération (3)) et verticalement (ou plus généralement perpendiculairement au plan dans lequel est disposé le patient).

Ce dispositif comprend essentiellement deux caissons (101 et 102) réalisés à partir de tôles pliées et soudées entre elles, qui sont fermés et ont pour but de rigidifier la fixation du dispositif selon l'invention. Le premier caisson (101) permet de fixer le support (1) en serrant deux vis à molette (105). Ce caisson est solidaire de deux écrous (103). Une vis (104), qui peut être actionnée manuellement ou même être motorisée, permet le mouvement du caisson (101) à l'intérieur du second caisson (102) lorsque l'on tourne la vis (104). Ceci permet de réaliser le mouvement vertical. L'originalité de ce dispositif réside dans la caractéristique que les deux caissons (101 et 102) coulissent avec un jeu important du fait que ceux-ci sont réalisés à l'aide de tôles pliées et soudées non usinées. Ce jeu sera annulé en solidarisant le tout de manière particulièrement rigide par le serrage des deux vis à molette (105) dans le support (1) lorsque la position verticale souhaitée est atteinte.

Le mouvement horizontal est réalisé à l'aide des deux écrous (109) solidaires du caisson (102). Une vis (110) permet de déplacer l'ensemble du support (1), solidaire des deux caissons (101 et 102), parallèlement au rail (2) de la table d'opération jusqu'à la position requise.

Un caisson (106) réalisé également en tôles pliées et soudées est prévu, et qui comprend deux clames (111) accrochées au rail (2) de la table d'opération, est également prévu. Des roulements (113) également fixés au caisson (102) coulissent sur une glissière (112) soutenant l'ensemble support (1) et des caissons (101 et 102) au cours du déplacement longitudinal. L'originalité de ce dispositif permet que lorsque l'on serre la vis (108), on clame le rail (2) entre les clames (111) et l'élément (107). Simultanément, on bloque les écrous (109) entre le caisson (106) et le même élément (107), annulant le jeu important de ces pièces non usinées et rendant ainsi tout l'ensemble particulièrement rigide.

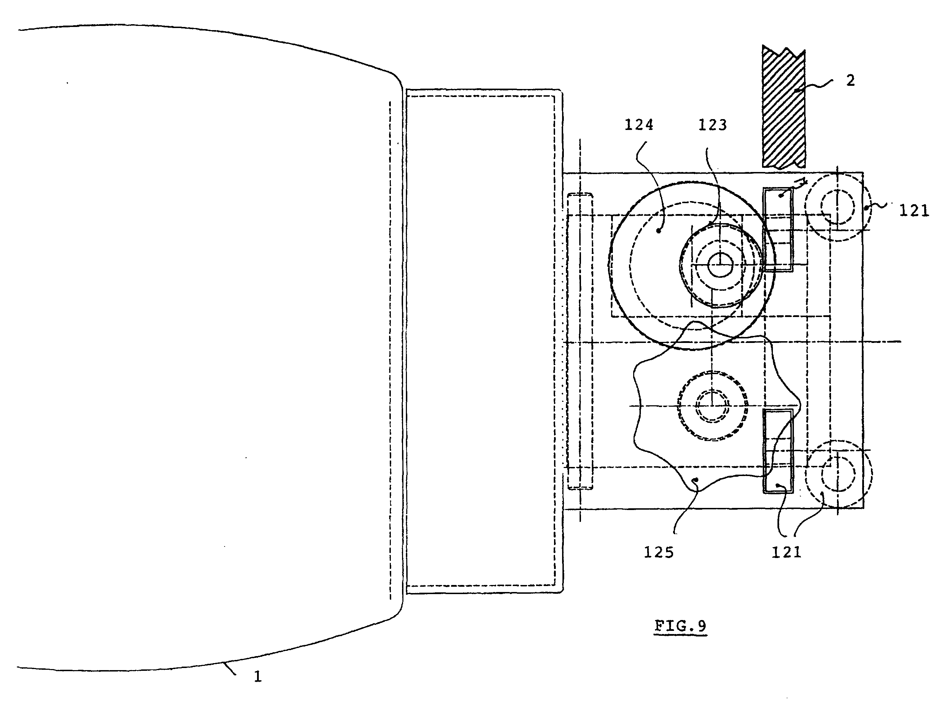

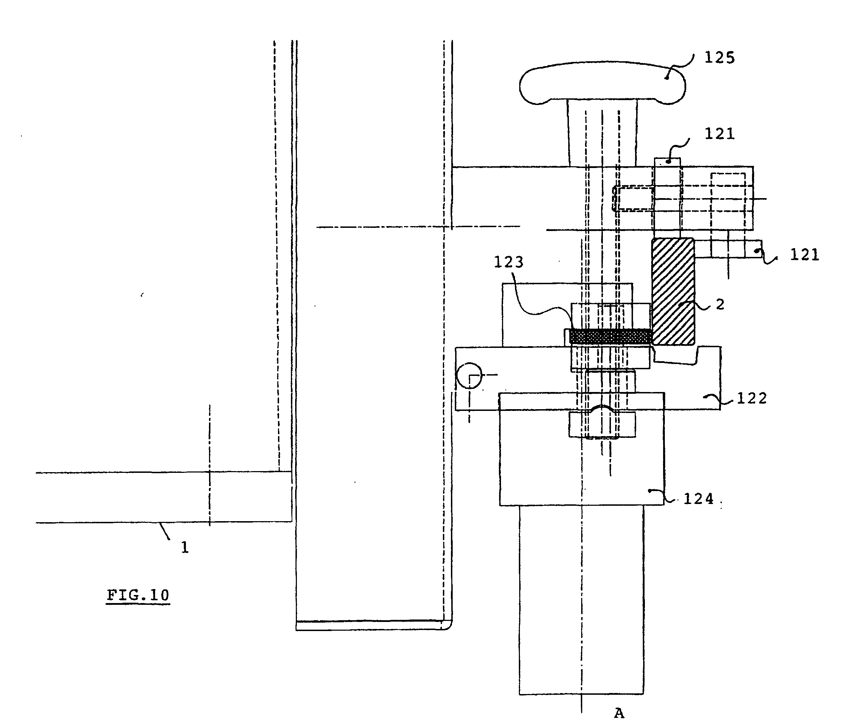

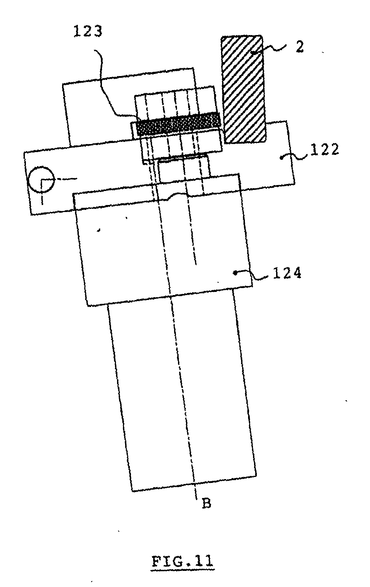

Selon une seconde forme d'exécution représentée plus en détails aux figures 9, 10 et 11, on utilise directement le rail (2) comme glissière en prévoyant quatre roulements orthogonaux (121) dans la partie haute du rail (2). Lorsque la clame inférieure (122) est libérée (en position A comme représenté à la figure 10), l'appui de la partie inférieure du rail (2) se fait directement sur une molette (123) actionnable par un motoréducteur (124). Cette molette (123) permet le déplacement le long du rail (2) . Lorsque la clame inférieure (122) est bloquée au moyen de la molette (125) et de sa vis (en position B comme représentée plus en détails à la figure 11), l'ensemble support (1) sera totalement immobilisé, le motoréducteur (124) et sa molette (123) étant dégagés.

## Revendications

1. Dispositif de manipulation d'un instrument chirurgical, comprenant un support fixe (1), un premier élément mobile (4) lié audit support fixe (1), un deuxième élément mobile (5) articulé à l'une des extrémités du premier élément mobile (4) et un dispositif d'accrochage (8) d'un instrument chirurgical (9), **caractérisé en ce que** :
- le premier élément mobile (4) est articulé au support fixe (1) autour d'un premier axe (H1) essentiellement parallèle au plan dans lequel est disposé le patient (PP') et susceptible d'effectuer un premier mouvement;
- le second élément mobile (5) est articulé à l'extrémité du premier élément mobile (4) autour d'un deuxième axe (H2) essentiellement parallèle au plan dans lequel est disposé le patient (PP') et susceptible d'effectuer un deuxième mouvement;
et **en ce qu'**il comprend en outre :
- un élément déformable (6) fixé par un côté à l'extrémité du deuxième élément mobile (5) et susceptible d'effectuer un troisième mouvement de rotation autour d'un troisième axe (C);
- un élément de préhension (7) fixé du côté opposé à celui du deuxième élément à l'élément déformable (6) et auquel est fixé le dispositif d'accrochage (8); et
- des moyens d'action (10 et 11) permettant d'effectuer des mouvements de rotation autour des différents axes (H1, H2 et C) .

2. Dispositif selon la revendication 1, **caractérisé en ce que** les premier (4) et deuxième (5) éléments mobiles se présentent sous forme de bras articulés, tandis que l'élément déformable (6) se présente sous la forme d'un quadrilatère déformable et articulé.

3. Dispositif selon la revendication 2, **caractérisé en ce que** le deuxième élément mobile (5) présente un point d'articulation qui peut coulisser dans une glissière (51) solidaire de l'élément déformable (6) tandis que l'autre point d'articulation peut être soit bloqué, soit libre.

4. Dispositif selon l'une quelconque des revendications précédentes, **caractérisé en ce que** l'élément de préhension (7) est disposé dans une glissière (71) solidaire de l'élément déformable (6).

5. Dispositif selon l'une quelconque des revendications précédentes, **caractérisé en ce que** l'élément support (1) est fixé à un rail (2) solidaire de la table d'opération (3) à l'aide d'un dispositif de fixation (100).

6. Dispositif selon la revendication 5, **caractérisé en ce que** le dispositif de fixation (100) comprend deux caissons (101 et 102) effectuant deux mouvements, un mouvement orthogonal au plan dans lequel est disposé le patient (PP') et un mouvement parallèle au plan du patient PP', le mouvement orthogonal étant réalisé à l'aide d'une vis (104) permettant l'introduction du premier caisson (101) dans le second caisson (102), le mouvement longitudinal étant réalisé à l'aide d'un troisième caisson (106) qui comprend des roulements (113) qui coulissent sur une glissière (112), la fixation du dispositif est réalisée lorsque l'on serre une ou plusieurs vis (108) en clamant le rail (2) entre les clames (111) et un élément adéquat (107).

7. Dispositif selon la revendication 5 ou 6, **caractérisé en ce que** le mouvement longitudinal est réalisé à l'aide de plusieurs, et de préférence quatre roulements (121) utilisant le rail (2) comme glissière, une clame inférieure (122) qui permet, au moyen d'une molette (125), d'immobiliser l'ensemble sur ledit rail (2).

8. Dispositif selon l'une quelconque des revendications précédentes 5 à 7, **caractérisé en ce que** le dispositif de fixation (100) est inversé par rotation autour d'un axe vertical et est ajusté par glissement le long du rail de fixation (26a) solidaire de la table d'opération (26).

9. Dispositif selon l'une quelconque des revendications précédentes, **caractérisé en ce que** le moyen d'accrochage (8) de l'instrument chirurgical (9) à l'élément de préhension (7) est constitué par un joint passif comprenant lui-même au moins deux pièces articulées selon deux axes perpendiculaires d'une part à l'axe de l'instrument chirurgical (9) et d'autre part à l'axe du premier élément (4), et dans lequel l'instrument chirurgical est fixé dans une position telle que le repère de verticalité de l'image dans le plan vertical soit assuré.

10. Dispositif selon l'une quelconque des revendications précédentes, **caractérisé en ce qu'**il comprend un dispositif de commande (50) constitué d'un mini-clavier (51) à au moins six touches (52) pour deux ou trois doigts, et dont la partie intérieure est de forme triangulaire.

11. Dispositif selon la revendication 10, **caractérisé en ce que** le dispositif de commande est complètement autonome et comporte un émetteur qui transmet les commandes au boîtier principal, cet émetteur se présentant sous la forme d'un petit boîtier de forme triangulaire.

## Patentansprüche

1. Vorrichtung zur Handhabung eines chirurgischen Instrumentes, die einen festen Träger (1), ein erstes bewegliches Element (4), das mit dem besagten festen Träger (1) verbunden ist, ein zweites bewegliches Element (5), das mit einem der Enden des ersten beweglichen Elementes (4) gelenkig verbunden ist, und eine Vorrichtung zum Aufhängen (8) des chirurgischen Instrumentes (9) aufweist, **dadurch gekennzeichnet, dass** :
- das erste bewegliche Element (4) mit dem festen Träger (1) um eine erste Achse (H1) gelenkig verbunden ist, welche im wesentlichen parallel zu der Ebene ist, in welcher der Patient (PP') angeordnet ist, und in der Lage ist, eine erste Bewegung durchzuführen;
- das zweite bewegliche Element (5) mit dem Ende des ersten beweglichen Elementes (4) um eine zweite Achse (H2) gelenkig verbunden ist, welche im wesentlichen parallel zu der Ebene ist, in welcher der Patient (PP') angeordnet ist, und in der Lage ist, eine zweite Bewegung durchzuführen;
und dass sie darüber hinaus enthält :
- ein verformbares Element (6), das mit einer seiner Seiten am Ende eines zweiten beweglichen Elementes (5) befestigt ist und in der Lage ist, eine dritte Rotationsbewegung um eine dritte Achse (C) durchzuführen;
- ein Greifelement (7), das an der Seite, die dem zweiten beweglichen Element gegenüber liegt, an dem verformbaren Element (6) befestigt ist, und an dem die Vorrichtung zum Aufhängen (8) befestigt ist; und
- Betätigungsmittel (10 und 11), die es ermöglichen, Rotationsbewegungen um die verschiedenen Achsen (H1, H2 und C) durchzuführen.

2. Vorrichtung gemäss Anspruch 1, **dadurch gekennzeichnet, dass** das erste bewegliche Element (4) und das zweite bewegliche Element (5) in der Form von Gelenkarmen vorliegen, während das verformbare Element (6) in der Form eines verformbaren Gelenkvierecks vorliegt.

3. Vorrichtung gemäss Anspruch 2, **dadurch gekennzeichnet, dass** das zweite bewegliche Element (5) einen Gelenkpunkt aufweist, der in einer Gleitschiene (51) verschoben werden kann, die mit dem verformbaren Element (6) fest verbunden ist, während der andere Gelenkpunkt entweder blockiert oder frei sein kann.

4. Vorrichtung gemäss irgendeinem der vorhergehenden Ansprüche, **dadurch gekennzeichnet, dass** das Greifelement (7) in einer Gleitschiene (71) angeordnet ist, die mit dem verformbaren Element (6) fest verbunden ist.

5. Vorrichtung gemäss irgendeinem der vorhergehenden Ansprüche, **dadurch gekennzeichnet, dass** das Trägerelement (1) an einer Schiene (2) befestigt ist, die mit dem Operationstisch (3) unter Zuhilfenahme einer Befestigungsvorrichtung (100) fest verbunden ist.

6. Vorrichtung gemäss Anspruch 5, **dadurch gekennzeichnet, dass** die Befestigungsvorrichtung (100) zwei Kasten (101 und 102) aufweist, welche zwei Bewegungen durchführen, bzw. eine Bewegung, die orthogonal zu der Ebene ist, in welcher der Patient (PP') angeordnet ist, und eine Bewegung, die parallel zu der Ebene des Patienten (PP') ist, wobei die orthogonale Bewegung mit Hilfe einer Schnecke (104) durchgeführt wird, welche die Einführung des ersten Kastens (101) in den zweiten Kasten (102) ermöglicht, wobei die Längsbewegung mit Hilfe eines dritten Kastens (106) durchgeführt wird, welcher Lager (113) aufweist, die sich auf einer Gleitschiene (112) verschieben, wobei die Befestigung der Vorrichtung dadurch verwirklicht wird, dass man eine oder mehrere Schrauben (108) anzieht, indem man die Schiene (2) zwischen den Spannpratzen (111) und einem geeigneten Element (107) fest spannt.

7. Vorrichtung gemäss Anspruch 5 oder 6, **dadurch gekennzeichnet, dass** die Längsbewegung mit Hilfe von mehreren, vorzugsweise von vier Lagern (121) unter Einsatz der Schiene (2) als Gleitschiene durchgeführt wird, wobei eine untere Spannpratze (122) es ermöglicht, das Ganze mit Hilfe eines Rändelrades (125) auf der besagten Schiene (2) festzuhalten.

8. Vorrichtung gemäss irgendeinem der vorhergehenden Ansprüche 5 bis 7, **dadurch gekennzeichnet, dass** die Befestigungsvorrichtung (100) durch Rotation um eine vertikale Achse umgekehrt wird und durch Gleiten entlang der Befestigungsschiene (26a) angepasst wird, die eine Einheit mit dem Operationstisch (26) bildet.

9. Vorrichtung gemäss irgendeinem der vorhergehenden Ansprüche, **dadurch gekennzeichnet, dass** die Vorrichtung zum Aufhängen (8) des chirurgischen Instrumentes (9) an das Greifelement (7) aus einer passiven Verbindungsstelle besteht, die ihrerseits mindestens zwei Teile aufweist, die nach zwei senkrechten Achsen gelenkig sind, einerseits zu der Achse des chirurgischen Instrumentes (9) und andererseits zu der Achse des ersten Elementes (4), wobei das chirurgische Instrument in einer solchen Position befestigt ist, dass die Markierung der Vertikalität des Bildes in der vertikalen Ebene gewährleistet ist.

10. Vorrichtung gemäss irgendeinem der vorhergehenden Ansprüche, **dadurch gekennzeichnet, dass** sie eine Betätigungsvorrichtung (50) umfasst, die aus einer Mini-Tastatur (51) besteht, die mindestens sechs Tasten (52) für zwei oder drei Finger aufweist, und deren innerer Teil eine dreieckige Form aufweist.

11. Vorrichtung gemäss Anspruch 10, **dadurch gekennzeichnet, dass** die Betätigungsvorrichtung vollkommen autonom ist und einen Sender enthält, der die Befehle an das Hauptgehäuse weiterleitet, wobei dieser Sender in der Form eines kleinen Gehäuses von dreieckiger Form vorliegt.

## Claims

1. A device for the handling of a surgical instrument, comprising a fixed support (1), a first movable element (4) linked to said fixed support (1), a second movable element (5) articulated on one of the ends of the first movable element (4) and a device for hanging (8) a surgical instrument (9), **characterized in that**:
- the first movable element (4) is articulated on the fixed support (1) around a first axis (H1) essentially parallel to the plane in which the patient is arranged (PP') and capable of executing a first movement;
- the second movable element (5) is articulated on the end of the first movable element (4) around a second axis (H2) essentially parallel to the plane in which the patient is arranged (PP') and capable of executing a second movement;
and **in that** it further comprises:
- a deformable element (6) fastened on one side to the end of the second movable element (5) and capable of executing a third rotational movement around a third axis (C) ;
- a gripping element (7) fastened on the opposite side to that of the second element to the deformable element (6) and to which the device for hanging (8) is fastened; and
- means of action (10 and 11) making it possible to execute rotational movements around the different axes (H1, H2 and C).

2. A device according to claim 1, **characterized in that** the first (4) and the second (5) movable elements take the form of articulated arms, while the deformable element (6) takes the form of a deformable four bar linkage.

3. A device according to claim 2, **characterized in that** the second movable element (5) has a point of articulation which can slide in a slideway (51) integral with the deformable element (6), while the other point of articulation can be either blocked or free.

4. A device according to any one of the preceding claims, **characterized in that** the gripping element (7) is arranged in a slideway (71) integral with the deformable element (6).

5. A device according to any one of the preceding claims, **characterized in that** the support element (1) is fastened to a rail (2) integral with the operating table (3), with the aid of a fastening device (100).

6. A device according to claim 5, **characterized in that** the fastening device (100) comprises two boxes (101 and 102) executing two movements, a movement othogonal to the plane in which the patient is arranged (PP') and a movement parallel to the plane of the patient PP', the orthogonal movement being carried out with the aid of a screw (104) allowing the first box (101) to be introduced into the second box (102), the longitudinal movement being carried out with the aid of a third box (106) which comprises rolling bearings (113) sliding on a slideway (112), the fastening of the device is carried out when one or more screws (108) are tightened by clamping the rail (2) between the clamps (111) and a suitable element (107).

7. A device according to claim 5 or 6, **characterized in that** the longitudinal movement is carried out with the aid of several, and preferably four rolling bearings (121) using the rail (2) as a slideway, a lower clamp (122) making it possible by means of a thumb wheel (125) to immobilize the assembly as a whole on said rail (2).

8. A device according to any one of the preceding claims 5 to 7, **characterized in that** the fastening device (100) is reversed by rotation around a vertical axis and is adjusted by sliding along the fastening rail (26a) integral with the operating table (26).

9. A device according to any one of the preceding claims, **characterized in that** the means for hanging (8) the surgical instrument (9) to the gripping element (7) consists of a passive joint which, for its part, comprises at least two components articulated along two axes perpendicular, on the one hand, to the axis of the surgical instrument (9) and, on the other hand, to the axis of the first element (4), and in which the surgical instrument is fastened in such a position that the verticality reference of the image in the vertical plane is ensured.

10. A device according to any one of the preceding claims, **characterized in that** it comprises a control device (50) which consists of a mini-keyboard (51) with at least six keys (52) for two or three fingers and the inner part of which is of triangular shape.

11. A device according to claim 10, **characterized in that** the control device is completely autonomous and comprises a transmitter which transmits the commands to the main case, this transmitter taking the form of a small case of triangular shape.
